# EUROPEAN PATENT APPLICATION

(11) **EP 3 789 015 A1**
(43) Date of publication of application: **10.03.2021**
(21) Application number: 19195674.7
(22) Date of filing: 05.09.2019
(51) Int. Cl.: A61K 9/20

(54) **FAST CONSOLIDATING COMPOUNDS**

(71) Applicant: Universität Innsbruck, 6020 Innsbruck (AT)
(72) Inventor: Griesser, Ulrich, 6094 Axams (AT); Schönemann, Magdalena Sonja, 78655 Dunningen (DE); Noisternig, Michael F., 6465 Nassereith (AT); Braun, Doris Elfride, 6020 Innsbruck (AT)
(74) Representative: Grünecker Patent- und Rechtsanwälte PartG mbB

(57) **Abstract**

The present invention relates to a process for the preparation of a dosage form comprising at least one fast consolidating compound and at least one active ingredient, a process for the preparation of a consolidated composition, a dosage form prepared by the process and the use of a mixture containing at least one fast consolidating compound and at least one solvent for 3D printing or another moulding process.

## Description

The present invention relates to a process for the preparation of a dosage form comprising a fast consolidating compound and an active ingredient, a process for the preparation of a consolidated composition, a dosage form prepared by the process and the use of a mixture containing a fast consolidating compound and a solvent for 3D printing.

The consolidation of a powder, e.g. for the preparation of dosage forms, is commonly performed by applying pressure (e.g. tableting) or using binders that form stable bridges between the powder particles. Such bridges can be formed in different ways by e.g. partly dissolving the surface with a solvent and subsequent drying, wetting with a binder solution and subsequent drying or by using a low melting binder that is dispersed in the powder at elevated temperature as a liquid and solidifies at ambient temperature.

The manufacture of oral solid dosage forms such as tablets is a complex multi-stage process. The most simple and economic way is direct tableting. The ingredients are mixed without previous granulation. Requirements therefore are sufficient plasticity of the mixture, good flowability and no tendency for segregation. Crystalline substances may show a broad plasticity range due to their structure features and relatively high pressures may be required to obtain a suitable tablet. Demixing is critical for low dose tablets. Even small variations in particle size or particle form may lead to tablets, which do not have the desired mechanical stability.

To improve process parameters and content uniformity tablets are very often produced from granules. Either wet granulation or dry granulation techniques are applied to produce such granules. Numerous unit processes are involved in making tablets, including particle size reduction and sizing, blending, granulation, drying, compaction, and (frequently) coating. Various factors associated with these processes can seriously affect content uniformity, bioavailability, or stability. The compression is done either by a single punch machine (stamping press) or by a multi station machine (rotary press).

The following problems may arise if any of the processes is not controlled properly:
- A possible change in solid state form of the active ingredient, rendering it less or totally inactive, or unstable.
- A change in particle size of the active compound and/or excipients, which may cause powder flow problems and particle segregation in the mix.
- Reducing the particle size can be very important for improving the dissolution rate of an API. However, this can implicate the formation of agglomerates, which may cause wettability and liberation problems.

All of these binding principles require either a drying process and/or elevated temperature and/or complex processing technologies. In particular, when elevated temperature is required, sensitive and thermally unstable active ingredients cannot be processed. Furthermore, often binders are used to provide stability for pharmaceutical dosage forms. Binders might affect the release characteristics of the active ingredient.

A consolidating compound is a compound, which, after contacting with a solvent, consolidates due to a phase transformation (solvation/hydration, crystallization, polymorphic transformation). A well-known consolidating compound is gypsum. Naturally occurring gypsum is calcium sulfate dihydrate (Ca₂SO₄ x 2 H₂O). When heated to about 110 °C the hemihydrate (calcined or burnt gypsum) is formed, and between 130 and 160 °C stucco plaster, a mixture of hemihydrate and anhydrate, is formed. Between 290 and 900 °C the anhydrate is formed and the crystal water is completely removed. Very high-heated gypsum is also called "dead-burnt gypsum" because it does not react with water any more. Upon addition of water, the hemihydrate becomes the dihydrate again, causing the material to harden or set in ways that are useful for casting and construction.

However, the solubility of gypsum in water is low and Ca₂SO₄ x 1/2 H₂O (burned gypsum) must be thoroughly mixed with the water to induce the formation of gypsum (Ca₂SO₄ x 2 H₂O) crystals. Without this mechanical mixing step burned gypsum is badly wettable and therefore it takes several ten minutes to hours to harden if water is added to burned gypsum powder without a thorough mixing process.

In contrast, the here disclosed concept offers a very fast consolidation reaction without a mixing step with a solvent (in contrast to burned gypsum), preferably within seconds to minutes, and allows the formation of consolidates without applying pressure or applying elevated temperature. This also allows to process very sensitive and thermally unstable active ingredients to a solid dosage form.

The problem underlying the present invention is the provision of processes for the preparation of dosage forms and consolidated compositions, which can be easily produced without requiring complex processing technologies, elevated temperature and/or additonal components.

Said problem is solved by a process for the preparation of a dosage form comprising at least one fast consolidating compound and at least one active ingredient, comprising the steps of (a) providing a mixture of at least one fast consolidating compound and at least one active ingredient, (b) adding at least one solvent to the mixture of step (a) to obtain a different solid form (cross-linking phase) of the fast consolidating compound, and (c) allowing the solid form of the fast consolidating compound of step (b) to consolidate to form the dosage form.

In an alternative, the problem of the present invention is solved by a process for the preparation of a fast consolidating composition, comprising the steps of (a) providing at least one fast consolidating compound, (b) adding at least one solvent to the fast consolidating compound of step (a) to obtain a different solid form (cross-linking phase) of the fast consolidating compound, and (c) allowing the cross-linking phase of the fast consolidating compound of step (b) to consolidate for less than 10 minutes, preferable less than 5 minutes.

The problem of the present invention is further solved by a dosage form obtainable by the process of the present invention.

In addition, the problem of the present invention is also solved by the use of at least one fast consolidating compound and at least one solvent for 3D printing.

Preferred embodiments of the present invention are set forth in subclaims 3 to 9, 11, and 13 to 15.

Surprisingly, we observed that specific solid forms of selected compounds show a very fast consolidation reaction after simple wetting with a liquid without mechanical mixing. Thus, the present invention provides a process, wherein compounds are used, which show a fast consolidation behavior after wetting with water or other solvents. This behavior is based on a very fast reaction (recrystallization) with the added solvent, preferably resulting in acicular shaped hydrate/solvate crystals. After contacting with a solvent, the fast consolidating compound consolidates due to a phase transformation (solvation/hydration, crystallization, polymorphic transformation) whereby the substance is partially dissolved and subsequently a recrystallization to a different solid form (cross-linking phase), mostly a solvated/hydrated form occurs. The solvent/water is incorporated into the crystal structure upon solvation, and the newly formed solvate/hydrate crystals result in crosslinking of the particles, particularly when the solvate/hydrate (or another polymorphic form) forms elongated crystals, such as needles.

The formation of a stable porous matrix within seconds to minutes can be used for the production of objects from powder samples without pressure (tableting) and is particularly useful for 3D printing technologies and the production of solid dosage forms containing active ingredients such as drugs or food supplements. The invention is also applicable for other processes that requires a fast consolidation procedure of a powder. Such processes can be utilized in the manufacturing of products in pharmaceutical industry, food industry or other industrial fields using toxicologically safe ingredients.

The present invention allows the selection, production and use of toxicologically safe, water soluble powder materials, which show a very fast consolidation after wetting with water or other solvents due to the formation of crystals of a different, mostly a solvated form (cross-linking phase). These materials can be used to quickly produce 3D models by using a mould or a 3D-printer.

The main advantage of the process of the present invention is the very short consolidation time, which cannot be achieved with binders nor with the addition of burned gypsum, which shows a very slow consolidation reaction. The process of the present invention can be used to form frameworks and bodies with other ingredients such as drugs and/or other fine chemicals or materials. The consolidated composition shows usually a high porosity, which is particularly advantageous if a fast dissolution is needed (drugs for example).

According to the present invention, the term "fast consolidating compound" is used for a compound, which, after wetting with a solvent, consolidates within short time, preferably 10 minutes or less.

The terms "consolidating" or "consolidate", according to the present invention, describes a cross-linking process, generally a process of recrystallization, of the respective compound after adding an amount of solvent that only partially dissolves the compound resulting in a "different solid state form" (named "cross-linking phase"), which can be a hydrate, a solvate with an organic solvent, a mixed solvate or another polymorphic form. The newly formed crystals crosslink and produce a network which can host other components.

The term "wetting" according to the present invention refers to a process of adding a solvent to a solid substance or a mixture of solid substances, wherein the solvent contacts the surface of the solid substance or mixture of solid substances.

The terms "non-toxic" or "not toxic" according to the present invention refers to compounds, which are approved and permitted in the field of pharmaceuticals and food products and are considered as having no toxic effect or only a low toxic effect on a subject.

According to the present invention the terms "active ingredient" and "active agent" are used interchangeably and refer to a compound having a specific effect or cause a specific reaction in an organism. Examples for active agents are pharmaceutically active agents, such as drugs. Alternatively, active agent may also include food additives, or other supplements.

The term "solvate" according to the present invention refers to a crystalline solid state form of an organic molecule or an inorganic salt, which hosts one or more solvent molecules in defined structural positions or voids. A "hydrate" is a specific form of solvate, wherein water is present as solvent in the structure of the host compound.

According to the present invention, the term "polymorphic transformation" refers to a change of the solid crystalline phase of a compound to a different solid phase of the same chemical composition but different crystal structure.

Food additives are substances added to food products to maintain or improve its safety, freshness, taste, texture, or appearance. Common food additives are colorants, preservatives, antioxidants, acid regulators, sweeteners, emulsifiers, stabilizers, thickening agents, gelling agents and flavoring agent.
Figure 1 shows the principle of the powder consolidation process through a phase transition.
Figure 2 shows an example of the process of the present invention for the formation of a dosage form.
Figure 3 shows the production of individualized tablets.
Figure 4 shows a scheme of the 3D powder printing process.

In a preferred embodiment in combination with any of the above or below embodiments, wherein the solvate crystals in step (c) of the process for preparation of a consolidated compound consolidate in less than 5 minutes, more preferably in less than 3 minutes, particularly preferably in less than 1 minute.

In a preferred embodiment in combination with any of the above or below embodiments the fast consolidating compound is a water-soluble compound, more preferably a compound having a solubility in water of more than 25 g/L at 20°C, most preferably more than 50 g/L, particularly preferably more than 200 g/L.

In another preferred embodiment in combination with any of the above or below embodiments, the fast consolidating compound is a carbohydrate anhydrate or an anhydrous salt or a partially dehydrated hydrate of the carbohydrate or salt, more preferably the fast consolidating compound is a partially dehydrated hydrate or an anhydrous form of α-lactose, raffinose, glucose, lactitol, calcium lactate, MgSO₄, Na₂SO₄ or Mg citrate. Particularly preferably the fast consolidating compound is α-lactose anhydrate, raffinose anhydrate or glucose anhydrate.

In a further preferred embodiment in combination with any of the above or below embodiments the fast consolidating compound is not burned gypsum.

The anhydrate can be produced by drying procedures known in the art, such as drying at elevated temperature. Preferably, the anhydrate is stable at temperatures in the range of 15 to 35°C and at a relative humidity of up to 80% to allow processing.

In a further preferred embodiment in combination with any of the above or below embodiments, the solvent is non-toxic.

In another preferred embodiment in combination with any of the above or below embodiments, the solvent preferably comprises water, alcohols, ether, esters, ketones, hydrocarbons, dimethylsulfoxide or a mixture thereof, more preferably the solvent is selected from water or a mixture of water and alcohol. The alcohol is preferably selected from methanol, ethanol, 1-propylalcohol, 2-propylalcohol, 1-butylalcohol or 2-butylalcohol, tert-butyl alcohol, 1-pentanol, iso-pentanol, tert-pentanol, 1,3-propanediol or mixtures thereof, more preferably ethanol. The ester is preferably selected from methylacetate, ethylacetate, propylacetate, or isobutylacetate, dimethyl carbonate or mixtures thereof. The ketone is preferable selected from acetone or methylethylketone. The hydrocarbon is preferably selected from hexane, heptane or pentane or mixtures thereof. Particularly preferably the solvent is water, ethanol or a mixture of water and ethanol.

In another preferred embodiment in combination with any of the above or below embodiments, water and alcohol are mixed in a weight ratio of 10 : 1 to 1 : 10 (water: alcohol), more preferably 5 : 1 to 1 : 5, most preferably 2 : 1 to 1 : 2.

In a particularly preferred embodiment in combination with any of the above or below embodiments, the solvent is water and ethanol in a weight ratio of 1 : 1.

In a further preferred embodiment in combination with any of the above or below embodiments, the solvent is added in an amount of 5 to 60 wt%, more preferably 10 to 50 wt%, particularly preferably 20 to 45 wt%, based on the total weight of the composition.

In a preferred embodiment in combination with any of the above or below embodiments, the weight ratio of fast consolidating compound to solvent is 1 : 10 to 5 : 1, more preferably 1 : 7 to 3 : 1, particularly preferably 1 : 5 to 1.5 : 1.

In another preferred embodiment in combination with any of the above or below embodiments, the composition further comprises a binder, more preferably the binder is selected from the group consisting of polyethylene glycol (PEG), cellulose ethers, sugars and sugar alcohols, starches or modified starches, polyvinylpyrrolidone (PVP) or polyvinylalcohol (PVA), gelatin, xanthan gum, carrageen, gum arabic, agar, guar gum, tragacanth and combinations thereof. The binder can be either added in step (a) of the process of the present invention or can be added to the solvent in step (b) of the process of the present invention.

In a further preferred embodiment in combination with any of the above or below embodiments, the binder is present in an amount of 0.1 to 10 wt%, more preferably 0.2 to 5 wt%, particularly preferably 0.5 to 1 wt%, based on the total weight of the composition.

The addition of a binder in the amounts allows the adjustment of the hardness of the final product.

In a preferred embodiment in combination with any of the above or below embodiments, the composition further comprises an additive selected from the group consisting of a flavoring agent, a colorant, a filler, a nutrient, a mineral supplement or a mixture thereof. The additive can be either added in step (a) of the process of the present invention or can be added to the solvent in step (b) of the process of the present invention. More preferably, the additive is present in an amount of 0.1 to 10 wt%, more preferably 0.2 to 5 wt%, particularly preferably 0.5 to 1 wt%, based on the total weight of the composition.

Furthermore, the additive can be a wetting agent to increase the wettability of the powder bed and ensure smooth surfaces. The additive can be either added in step (a) of the process of the present invention or can be added to the solvent in step (b). More preferably, the additive is present in an amount of 0.01 to 10 wt%, more preferably 0.2 to 5 wt%, particularly preferably 0.5 to 1 wt%, based on the total weight of the composition.

In a preferred embodiment in combination with any of the above or below embodiments, the active ingredient is a food supplement, more preferably a vitamin, mineral supplement, amino acid, essential fatty acid, fibre, or plants or herbal extracts or a mixture thereof.

In another preferred embodiment in combination with any of the above or below embodiments, the active ingredient is a pharmaceutically active ingredient, more preferably an analgesic, a cholesterol-lowering medicament, an agent for treating hypertension, an agent for treating diabetes, an agent for treating cardiac conditions, an antihistamine, a hormone or hormone inhibitor, an enzyme inhibitor, an anticancer agent, an antiepileptic, an antiinfective, such as antibiotic, antiviral agent, antimycotic agent, an antiemetic, a laxative, an antithrombotic agent, an immunostimulant, an immunosuppressive, an antirheumatic, a muscle relaxant, a psycotropic drug or a mixture thereof. Particularly preferably the pharmaceutically active ingredient is selected from the group consisting of paracetamol, ibuprofen, diclofenac. levetiracetam, aspirin, metamizol, hydrochlorothiazide, amiloride, captopril, warfarin, dicoumarol, prednilolone, dexamethasone, aripiprazole, risperidone, levothyroxine, tacrolimus, ramipril, perindopril, codeine, atorvastatin, rifaximin, sofosbuvir, bisoprolol, nebivolol, telmisartan, sildenafil, loratadin, lamotrigine, folic acid, rucaparib, vancomycin, ofloxacin, theophylline, caffeine, paclitaxel, fentanyl, morphine, buprenorphine, tramadol, naloxone, sufentanil, levodopa, odansetrone, dimehydrinate, donepezil, ticagrelor, pridopidine, baricitinib, zonisamid, asenapin, vardenafil, tetracycline, mirtazapin, rivastigmin or a mixture thereof.

In a preferred embodiment in combination with any of the above or below embodiments, the active ingredient is present in the composition of the present invention in amount of 5 to 90 wt%, more preferably 20 to 80 wt%, particularly preferably 40 to 70 wt%, based on the total weight of the composition.

In another preferred embodiment in combination with any of the above or below embodiments, the active ingredient is present in the composition of the present invention in amount of 0.01 to 5 wt%, more preferably 0.5 to 3 wt%, particularly preferably 1 to 2 wt%, based on the total weight of the composition.

In a preferred embodiment in combination with any of the above or below embodiments, the dosage form of the present invention has an API load of at least 40 wt%, more preferably at least 50 wt% and particularly preferably at least 60 wt%, based on the total weight of the consolidated compound. Thus, the process of the present invention allows the formation of dosage forms having a high API (active pharmaceutical ingredient) load.

In another preferred embodiment in combination with any of the above or below embodiments, the dosage form of the present invention has an API load 0.01 to 5 wt%, more preferably more preferably 0.5 to 3 wt%, particularly preferably 1 to 2 wt%, based on the total weight of the consolidated compound. Dosage forms having a low API load are particularly useful for APIs having a narrow therapeutic range. Such dosage forms are preferably prepared by adding the API to the powder bed or dissolving the API in the solvent.

In another preferred embodiment in combination with any of the above or below embodiments, the fast consolidating compound is not toxic and/or edible.

In a preferred embodiment in combination with any of the above or below embodiments, the solvent in step (b) is added at a temperature of 0 to 60°C, more preferably 10 to 40°C, most preferably 15 to 30°C. In a particularly preferred embodiment the solvent is water or a mixture of water and ethanol and is added at a temperature of 10 to 25°C, in an amount of 10 to 40 wt%, based on the total weight of the composition.

In a further preferred embodiment in combination with any of the above or below embodiments, the morphology of the newly formed solid form of the fast consolidating compound is acicular (needle shaped), platy or prismatic, more preferably the particles are needle-shaped. The particle shape of the cross-linking phase of the fast consolidating compound allows the formation of frameworks, particularly with high porosity, which allows fast disintegration and is therefore particularly useful for drugs as active ingredient.

Dosage forms according to the present invention are pharmaceutical drug products or food products in the form in which they are marketed for use, with a specific mixture of active ingredients and inactive components (excipients), in a particular configuration (such as a capsule or shell), and apportioned into a particular dose.

The present invention provides a dosage form obtainable by the process of the present invention. In a preferred embodiment the dosage form is a tablet, capsule, lozenge, pastille, granule, sachet, reconstitutable powder, powder, dry powder inhaler or chewable.

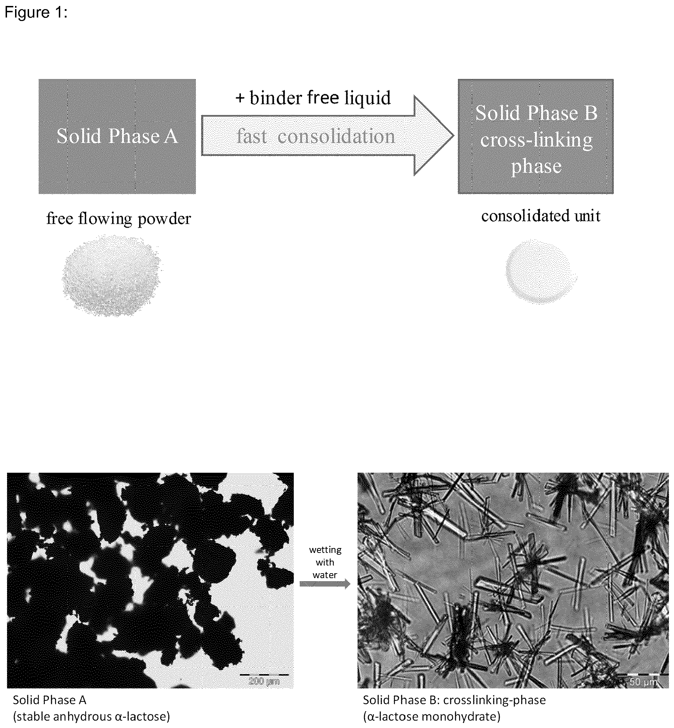

In a preferred embodiment in combination with any of the above or below embodiments, the process of the present invention is used for the preparation of a solid dosage form by moulding. In a particularly preferred embodiment the mixture of the fast consolidating compound and the active ingredient is placed in a mould, the solvent is added and after consolidation the consolidated compound is removed from the mould. More preferably, the mould is sealed with a film to provide the final dosage form directly in a primary pack (Figure 1).

Advantages of a moulding process are:
- simple production process;
- fast consolidation within seconds to minutes;
- short time required for drying, since part of the solvent is usually incorporated into the structure of the solid;
- less stress, no high temperatures or pressure required;
- economic process for the preparation of dosage forms; and
- preparation of individualized dosage forms
- providing the final dosage form directly in the primary pack.

In another preferred embodiment in combination with any of the above or below embodiments, the fast consolidating compound can be used for 3D printing.

In a 3D printing process material is joined or solidified under computer control to create a three-dimensional object, with material being added together (such as liquid molecules or powder grains being fused together), typically layer by layer.

In the present invention at least one fast consolidating compound and at least one solvent are used for 3D printing.

In a preferred embodiment in combination with any of the above or below embodiments, in the 3D printing process the fast consolidating compound is contacted with the solvent and thereby solidified and a first layer of the 3D object is formed. The step is repeated until the desired product is formed.

Advantages of the 3D printing process are:
- fast consolidation after addition of solvent and thus very fast 3-D printing rates possible
- short/no drying time due to the incorporation of the solvent in the fast consolidating compound. The drying time of excess solvent depends on the volatility of the used solvent;
- Very low or very high API loads in the solid dosage form possible;
- no stress due to temperature and pressure.

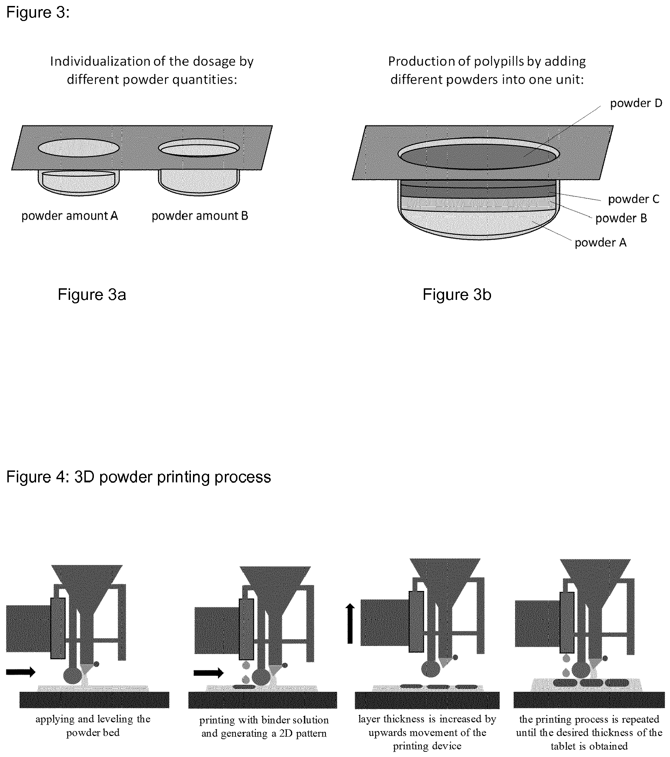

The present invention further allows the provision of individual dosages of the active ingredient and/or to combine different active ingredients in one tablet. In a preferred embodiment in combination with any of the above or below embodiments, a dosage form can be formed by mixing at least one fast consolidating compound with different amounts of the active ingredient per tablet and a multi-layer dosage form wherein each layer contains a different concentration of the active agent can be formed (Figure 3a). In a preferred embodiment, the dosage of the active ingredient per tablet can be individualized by adding different powder quantities (moulding process) or by printing different amounts of layers (3D-powder printing). In another preferred embodiment in combination with any of the above or below embodiments, a multi-layer dosage form comprising different active ingredients in one tablet can also be formed by adding different excipient/API mixtures into one unit (Figure 3b).

For the production, the multi-layer dosage form can be produced layer by layer, i.e. by repeating steps (a) to (c) for each mixture of fast consolidating compound and active ingredient. Specifically, steps (a) to (c) are carried out for the first mixture of fast consolidating compound and active ingredient, then the steps are repeated for the second mixture, and then the steps are again repeated for the third mixture. The steps are repeated until the desired dosage form is finished. The multi-layer dosage form can contain layers with different active agents and/or active agents in different concentrations. Furthermore, the multi-layer dosage form can be formed in a manner to provide controlled disintegration at a specific site of action, e.g. by selecting the fast consolidating agent. This process is preferably used for 3D printing but can also be used in the moulding process.

Alternatively, the dosage form can be produced in one step by placing a first layer of a mixture of fast consolidating compound and active agent in a form and then placing a second layer of a mixture of a fast consolidating compound and active agent above the first layer. Again, the steps are repeated until the desired layers of powder mixture have been applied. Then the solvent is added to form the multi-layer dosage form. This process is preferably used in the moulding process. The moulding process is further suitable for producing tablets, wherein each layer contains a different concentration of the active agent (Figure 3a).

In the present invention the formation of the crosslinking-phase (in most cases solvate crystals) and the consolidation occurs rapidly, much faster than the consolidation of gypsum. The fast consolidating compounds are preferably water-soluble and in the processes of the present invention no mixing is required. It is sufficient to wet the fast consolidating compound with the solvent.

The following examples further describe the present invention.

### Examples

### 1. General

All percentages given in the examples refer to wt%, unless noted otherwise. The solutions described herein are aqueous solutions unless noted otherwise.

Abbreviations:
- AH: anhydrate
- MH: monohydrate
- EtOH: ethanol
- 2-PrOH: 2-propanol
- Lα_{S}: stable α-Lactose anhydrate
- PVA: polyvinylalcohol
- PEG: polyethylene glycol

### 2. Preparation of fast consolidating materials

The anhydrates of the tested compounds were prepared from the respective hydrates under the conditions as shown in Table 1 below.

**Table 1: Preparation of fast consolidating materials (examples)**

| **Example** | **Hydrate form (starting material)** | **Anhydrate production** | **Solid form used for fast consolidation process** |
|---|---|---|---|
| **1** | α-Lactose monohydrate | drying at 150 - 160°C or refluxing in EtOH at 85°C | α-Lactose anhydrate |
| **2** | Raffinose pentahydrate | drying at 100 - 110°C | Raffinose anhydrate |
| **3** | Glucose monohydrate | drying at 75 °C | Glucose anhydrate |
| **4** | Ca-Lactate pentahydrate | drying at 70 - 125°C | anhydrous Ca-Lactate |
| **5** | MgSO₄ hepta-/hexahydrate | drying at 110 - 150°C, rest of water at 300°C | MgSO₄ anhydrate |
| **6** | Na₂SO₄ decahydrate | drying at 30°C | Na₂SO₄ anhydrate |
| **7** | Lactitol MH | drying at 105 °C | Lactitol anhydrate |
| **8** | Mg Citrate Dodecahydrate | drying at 180 °C | anhydrous Mg Citrate |

### 3. Moulding process

### 3.1 α-Lactose anhydrate

### 3.1.1 Influence of the solvent on the formation of stable tablets

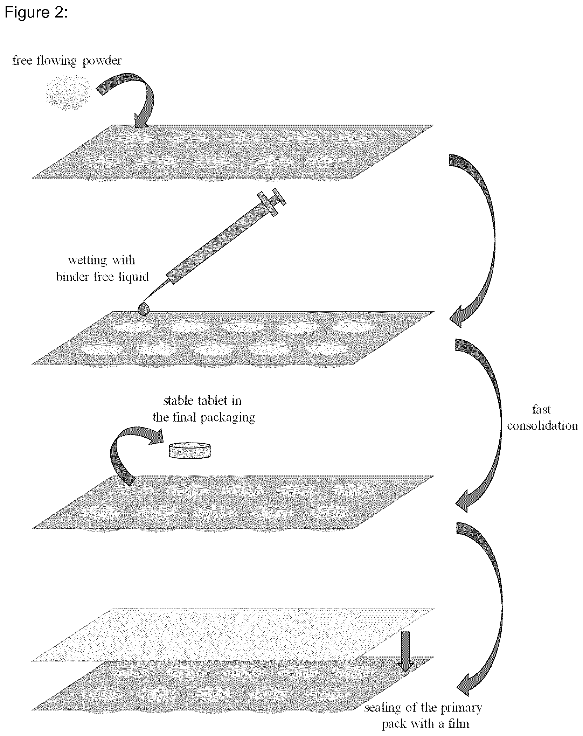

α-Lactose anhydrate was transferred into wells of a moulding form and wetted with pure water and water/alcohol mixtures, as shown in Figure 2. Pure water and aqueous EtOH solutions, 25% (v/v) and 50% EtOH (v/v), ratio by volume, were used as solvents. These experiments were performed to measure the time needed for the formation of stable tablets, to determine the optimal amount of solvent, and to determine the optimal water:alcohol ratio in water/alcohol mixtures.

Wetting with pure water results in stable tablets within 5 - 10 seconds, whereas in the case of water/alcohol mixtures more than 10 seconds are required to produce stable tablets. The tablets could be removed from the moulding form after a few seconds when wetted with pure water.

The best results were achieved by adding 50 - 60 % water with regard to the powder mass (µl liquid to mg powder). This means that about 125 - 150 µl of water are needed for a 250 mg tablet. With the aqueous ethanol solutions, the amount of solvent had to be increased in order to sufficiently wet the entire powder bed. Here an amount of 90 - 100 % liquid relative to the powder mass is needed.

### 3.1.2 Influence of the starting material (α-Lactose) on tablet properties

For the production of tablets with the moulding process, it is extremely important to wet the entire powder bed homogenously. Granulac 70 monohydrate (a-Lactose monohydrate, Meggle), Spherolac 100 monohydrate (a-Lactose monohydrate, Meggle), Prismalac 40 (α-Lactose monohydrate, Meggle) and Granulac 230 (a-Lactose monohydrate, Meggle) were used as starting materials. EtOH solutions with different amounts of polyvinylalcohol PVA 388 (Kuraray) were used as binder. Granulac 70 and Spherolac 100 resulted in uniform tablets, whereas Prismalac 40 and Granulac 230 produced incomplete tablets because the binder solvent did not intrude evenly into the powder bed.

In the next step, the influence of the solid form [monohydrate (MH) and dehydrated powder (Lαₛ, AH)] and the influence of binder was investigated for Spherolac 100 and Granulac 70. The tablets produced using the MHs as starting substance require binders (PVA) and long drying times, because solidification only occurs if complete solvent evaporation takes place (no fast consolidating process). The obtained tablets were uniform, but drying took several hours. Spherolac 100 showed the most uniform wetting. The best tablets were obtained with dehydrated Spherolac 100 (AH) and 5 wt% PVA in water or in 50% EtOH (v/v) in a ratio of 90 - 100 % (µl binder solution to mg powder), thus, through a fast consolidating process.

The tablet hardness was measured with a three-point-break tester by Charles Ischi AG (HC 6.2 50 N).

The tablets produced with Lα_{S} (AH) as starting material resulted in harder tablets than those produced with LαMH and a binder (Table 2). It is important to note that the tablets with pure water as solvent, without the addition of a binding agent, achieve hardnesses comparable to the tablets with binding agents in the liquid. This shows that it is not necessary to add a binder to the solvent.

**Table 2: Tablet hardness of α-Lactose tablets.**

| **Starting material** | **Amount [mg]** | **Binder solution** | **Average hardness of tablet [N]** |
|---|---|---|---|
| Spherolac 100 AH | 200 | water | 9.4 |
| Spherolac 100 AH | 200 | 5 wt% aq. PVA 388 | 8.65 |
| Spherolac 100 AH | 200 | 7.5 wt% aq. PVA 388 | 12.2 |
| Spherolac 100 AH | 200 | 10 wt% aq. PVA 388 | 14.75 |
| Spherolac 100 AH | 200 | 5 wt% PVA 388 in 50% EtOH (v/v) | 10.45 |
| Spherolac 100 MH | 200 | 5 wt% PVA 388 in 50% EtOH (v/v) | 5.85 |
| Spherolac 100 AH | 200 | 7.5 wt% PVA 388 in 50%EtOH (v/v) | 16.75 |
| Spherolac 100 MH | 200 | 7.5 wt% PVA 388 in 50% EtOH (v/v) | 8.1 |
| Spherolac 100 AH | 200 | 10 wt% PVA 388 in 50% EtOH (v/v) | 20.2 |
| Spherolac 100 MH | 200 | 10 wt% PVA 388 in 50% EtOH (v/v) | 13.1 |

The experiment was repeated with 2-propanol, instead of ethanol, and the results were the same as with ethanol: dehydrated Granulac 70 (AH) and dehydrated Spherolac 100 (AH) with 5 wt% PVA in water or in 50 % aqueous 2-PrOH (v/v) in a ratio of 90% (µl to mg) resulted in the best tablets. The difference between ethanol and 2-propanol is the faster evaporation of 2-propanol.

### 3.1.3 Influence of binders on tablet properties

Polyvinylalcohol (PVA) 388 and PVA 398 were tested. PVA 398 has a higher viscosity (3.2 - 38 mPa x s) and hydrolysis grade than PVA 388. PVA 398 has a higher binding effect and can therefore be used in lower concentrations and quantities. PVA 388 is more suitable because of the simpler preparation process of the solutions.

Additionally, PEG 3000 was tested as binder, which shows a high solubility in water. The polymer solution was applied on dehydrated Spherolac 100 (AH) as 10 wt% and 4 wt% aqueous solutions. The optimal result was obtained with 10 wt% aqueous PEG 3000 solution. The tablets show a smooth, wax-like surface. An advantage compared to PVA 388 is the easier production of the binder solution. Though, PVA shows a clearly higher binding strength, which results in harder tablets than with PEG.

### 3.1.4 Influence of anhydrate production on tablet properties

The in section 3.1.3. described experiments were carried out with anhydrates (AH) produced by the refluxing method (Lαₛ₋Rfx) and oven drying method (Lαₛ). The oven dried Lαₛ show a better powder flow behavior and does not agglomerate. This results in a better wettability and tablets with smoother surfaces. Thus, the preparation process chosen for preparing of a fast consolidation material significantly influences the quality of tablet obtained through fast consolidating. An improved production method, oven drying, of the anhydrate was developed.

### 3.2 Glucose anhydrate

Anhydrous glucose was placed into a moulding form and different solutions were dispensed onto the powder. Because of the high water solubility of anhydrous glucose a significantly lower amount of binder solution (ca. 30 µl water for 100 mg powder) is required in contrast to anhydrous α-lactose. Though, the wettability is worse than that of α-lactose.

### 3.2.1 Influence of solvent composition of solidification time

The solidification time (time until the tablets show sufficient hardness for handling) after addition of the solvent was determined using pure water and an aqueous glucose solution. It was observed that the solidification time of anhydrous glucose is much shorter when glucose solutions are applied instead of pure water. Glucose anhydrate solidifies after 10 - 15 min when water is used but hardens within 2 - 6 minutes after the application of glucose solutions. The amount of glucose in the solution is less critical for the solidification time, a concentration of 25 wt% is sufficient.

### 3.3 Raffinose anhydrate

### 3.3.1 Extending the fast consolidation time (to minutes) of a compound with a poor wettability

The moulding form tests were carried out with two different solvents: a) water and b) 50% EtOH (v/v) solution. If the powder is moistened with water, it becomes solid within a few seconds. To increase the poor wettability of the material a 50 % EtOH (v/v) solution was tested, resulting in an extension of the consolidation period to about four to five minutes.

### 3.4 Ca-Lactate anhydrate

In a first experiment, tablets with amorphous Ca-L-Lactate (L-CaLac) using pure water were produced. The flowability of amorphous L-CaLac is excellent and comparable with dehydrated Spherolac 100 (AH). The material shows a good wettability and within one minute, the tablets consolidate and can be removed from the moulding form. A solvent/powder ratio of 40% proved to be optimal and resulted in the best tablets.

### 3.5 MgSO₄

### 3.5.1 Extending the fast consolidation time (to minutes) of a compound showing a fast and very exothermic consolidation reaction

Using completely water free MgSO₄ resulted in a solidification within a few seconds after wetting with pure water. The powder/solvent (water) ratio was 50 - 60%. However, the consolidation reaction is very exothermic.

Wetting partly anhydrous MgSO₄(7 wt% water content) with pure water results in solid tablets. After 5 minutes the tablets can be unbaged from the moulding form. The wettability is not as good as for the α-lactose powders. About 50 - 60 µl water were added per 100 mg powder. By using a saturated aqueous solution of MgSO₄ the hardening time could be halved (2 - 3 minutes).

Thus, using partially hydrated MgSO₄ is preferred as it shows slower transformation rates (few minutes).

### 3.6 Na₂SO₄ anhydrate

It needs about 18 µl water for 100 mg of the well wettable and freely flowable powder. The tablets were solid within a few seconds and could be removed from the moulding form. To enable an API loading via the wetting liquid, the sodium sulfate powder was wetted with a 50% EtOH (v/v) solution. Mechanically stable tablets were obtained within one minute.

### 3.6.1 Increase of the stability of an unstable hydrate

Attempts were undertaken to ensure the permanent stability of the Na2SO4 (decahydrate) tablets by adding binder polymers to the wetting liquid. Amounts of 5 wt% and 7.5 wt% aqueous PVA 388 solutions were tested. The tablets wetted with the 5 wt% PVA 388 solution were considerably more stable the next day than the tablets produced with pure water as liquid, but still could be easily crushed. The 7.5 wt% PVA 388 solution produced mechanically stable tablets after drying for two and a half days (stored at 22°C or 40°C). The tests show the possibility of a new approach: rapid solidification by hydrate formation and permanent stability through additional binders.

Furthermore, a mixture of 50 wt% Na₂SO₄ anhydrate and 50 wt% glucose anhydrate was wetted with a 25 wt% glucose solution. The tablets could be removed after a few seconds and the permanent stability was reached without a polymer. This excipient mixture has two advantages: firstly a faster solidification of anhydrous glucose and secondly a permanent stability of the tablet.

### 3.7 Mg Citrate anhydrate

Wetting Mg Citrate anhydrate powder, containing a small amount of seed crystals of the dodecahydrate (about 2 %), with pure water results in stable tablets after 1 to 4 min. Without seed crystals the solidification time is about 4 min. The wettability can be increased with wetting agents.

**Table 3: Overview of the powder/liquid ratio and the hardening time**

| **Solid form used for fast consolidation** | **Solution** | **Powder/liquid ratio (µl liquid to mg powder)** | **Consolidation time** |
|---|---|---|---|
| Stable α-Lactose anhydrate | water | 50 - 60% | 5-10 seconds |
| Raffinose anhydrate | water | ca. 30% | few seconds |
| Glucose anhydrate | water | 30% | ca. 10 min |
| | Aqueous glucose solution (25 - 50 wt%) | 30% | 2-6 min |
| Ca-Lactate anhydrate | water | 35 - 40% | 1 to 2 min |
| MgSO₄ + 7 wt% H₂O | water | 50 - 60% | ca. 5 min |
| MgSO₄ anhydrate | water | 50 - 60% | few seconds |
| Na₂SO₄ anhydrate | water | ca. 15% | few seconds |
| Mg Citrate anhydrate | water | 40 - 50 % | 1 to 4 min |
| Calcium sulphate hemihydrate (burned gypsum) | water | 100% | ca. 35 min |

### 4. Tests of the drug load capacity of the tablets produced by the moulding process

As model substance for the drug load capacity test, Spheriglass 2429 (glass beads, Potters Europe) was used, because there are no interactions between the glass beads and lactose to exclude other effects. Spheriglass 2429 has a particle size distribution of 53 - 106 µm. It was mixed with refluxed and sieved Spherolac 100 anhydrate (Spherolac 100 AH) (< 250 µm) in a ratio of 30 and 60 wt%. All tablets were stable within a few seconds after wetting and were mechanically stable for handling. The higher the amount of Spheriglass 2429 the less binder solution is needed, because of the decreasing amounts of water binding excipients.

A mixture of 50 wt% anhydrous glucose and 50 wt% Spheriglass 2429 was wetted with a 25 wt% glucose solution and pure water. The aim of this experiment was to investigate if the consolidation time is slowed down by loading a slower solidifying hydrate (in comparison to a-Lactose anhydrate) with a non-matrix-forming substance. After four minutes the tablets could be removed but after 10 minutes they were still rather soft. The advantages of the use of glucose are rather seen as additive to other fast consolidating excipients to improve or modify the consolidation process, its rate, and the properties of the product.

It was observed that the addition of Spheriglass improved the wettability of anhydrous raffinose. Two minutes after wetting with pure water the tablets consolidated and could be removed from the moulding form. With a 50% EtOH (v/v) solution, the tablets could be also removed after 2 - 4 minutes and were more stable and more uniform in shape than the tablets produced with pure water. This indicates that the wettability and also the flowability of the consolidating excipient can be improved significantly by using suitable additives.

A loading of 50 wt% Spheriglass 2429 with anhydrous Ca-L-lactate produces stable and uniform tablets with water as wetting liquid. A sufficient mechanical stability was also observed for tablets produced with a 50% EtOH (v/v) binder solution, though the solidification takes much longer as with pure water (ca. 45 min).

A mixture of 50 wt% Spheriglass 2429 and 50 wt% MgSO₄ (water content about 7 wt% H₂O) became solid within five minutes, whereas MgSO₄ anhydrate with Spheriglass 2429 led to stable tablets after a few seconds. Furthermore, the hydrate formation was less exothermic than without Spheriglass 2429.

A mixture of 50 wt% Spheriglass 2429 and 50 wt% Na2S04 anhydrate wetted with water yielded stable tablets within a few seconds.

An amount of 50 % wt of Spheriglass 2429 added to Mg Citrate anhydrate leads to stable tablets after about 10 min or more, after wetting with water. The wettability is significantly increased by the addition of Spheriglass 2429.

**Table 4: Load capacity and curing times of mixtures of different fast consolidating materials with glass beads.**

| **Substance** | **Loading capacity** | **solvent** | **Consolidation time** |
|---|---|---|---|
| Stable α-Lactose anhydrate | 60% | H₂O H₂O / EtOH 1 : 1 | few seconds few seconds |
| Glucose anhydrate | 50% | H₂O | > 10 min |
| Raffinose anhydrate | 50% | H₂O H₂O / EtOH 1 : 1 | 2 min 4 min |
| Ca-Lactate anhydrate | 50% | H₂O | 1 to 2 min |
| MgSO₄ + 7 wt% H₂O | 50% | H₂O | ca. 5 min |
| MgSO₄ anhydrate | 50% | H₂O | few seconds |
| Na₂SO₄ anhydrate | 50% | H₂O | few seconds |
| Mg Citrate anhydrate | 50% | H₂O | > 10 min |

### 5. 3D powder printing tests

In the 3D printing process a thin layer of a fast consolidating compound and optionally an active agent is placed onto a glass plate and leveled with a spinning roller. The solvent for consolidating the powder particles is applied onto selected areas of the powder using inkjet technology. Then a new layer of powder is spread out followed by another wetting step (Figure 4). This process is repeated until for example a tablet is formed.

### 5.1 Printing tests and adjustment of print parameters

Stable α-Lactose anhydrate was produced from Spherolac 100 MH by drying in a drying oven. The resulting Spherolac 100 AH was then used for 3D printing tests. The material was sieved (180 |jm mesh) to ensure that all particles will pass through the integrated sieve of the printing device. Granulac 230 (Meggle) was a further component of the powder bed. Granulac 230 MH was also dried in a drying oven. As a model substance Spheriglass 2429 (Potters Europe) was used. In order to make the surface of the tablets smoother and to reduce abrasion a mixture of Spherolac 100 AH and milled PEG 3000 was also tested. The following substance/binder combinations (Table 5) were used to find optimized parameters.

**Table 5: Substance/liquid combinations of the performed printing tests.**

| **Spherolac 100 AH amount in wt%** | **Granulac 230 AH amount in wt%** | **Spheriglass 2429 amount in wt%** | **PEG 3000 amount in wt%** | **Liquid** |
|---|---|---|---|---|
| - | 100 | - | - | H₂O |
| 100 | - | - | - | H₂O |
| 50 | 50 | - | - | H₂O |
| 70 | 30 | - | - | H₂O |
| 85 | 15 | - | - | H₂O |
| 95 | 5 | - | - | H₂O |
| 67 | 10 | 23 | - | H₂O |
| 75 | - | 25 | - | H₂O |
| 100 | - | - | - | EtOH 15% (w/w) |
| 85 | 15 | - | - | EtOH 15% (w/w) |
| 70 | - | 30 | - | EtOH 15% (w/w) |
| 90 | - | - | 10 | EtOH 15% (w/w) |

Granulac 230 is a milled product. As a result, the flowability was low and the water did not penetrate completely or only slowly into the powder bed. These circumstances led to a longer solidification time and a longer transformation time of the anhydrate. Spherolac 100 AH has a very good flowability and the solidification was fast. Cohesiveness was more the problem here. During the printing process, the printed objects began to slide back and forth on the printing surface. This problem could be solved by adding a portion of cohesive powder (e.g. Granulac 230) or by adjusting the printing parameters. A mixture of Spherolac 100 AH and Spheriglass 2429 further increased the flowability due to the round particles of Spheriglass, which filled the spaces of Spherolac 100 AH and thus led to a denser packed tablet. Besides pure water, a 15% EtOH solution (w/w) was tested as liquid. The alcohol content led to an increased wettability between the liquid and the powder. The tablets with PEG 3000 still had a high abrasion.

Square and round shapes were printed. The 3D-models were designed with Autodesk Fusion 360. One approach was the printing of continuous tablets, which can then be broken into single doses. The tablets could be handled directly after the printing process and were stable. In addition, the active ingredients in this process are not exposed to temperature and pressure stress.

### 5.2 Analyses of the printed tablets

### 5.2.1 Uniformity of tablets

For this test three different batches of rectangular tablets (10 x 10 x 5 mm) were printed. The powder bed of batch 1 consisted of Spherolac 100 AH (<180 µm). The powder bed of batch 2 was a mixture of 50 wt% Spherolac 100 AH (<180 µm) and 50 wt% Granulac 70 MH(<180 µm) as model substance. Batch 3 was a mixture of 44 wt% Spherolac 100 AH (<180 µm), 44 wt% Granulac 70 MH (<180 µm), 10 wt% sucrose (<125 µm) and 2 wt% silicon dioxide. Sucrose is added as another binder and silicon dioxide is added for a better flowability. The liquid for all three batches was a 15% EtOH (w/w) solution. The sucrose tablets were first gel-like and deformable, but the final hardness was better, the tablets were more uniform and showed less abrasion. The dimensions were measured with a micrometer (Mitutoyo - coolant proof micrometer IP65). The dimensions showed only small deviations and there were no significant differences between the batches. Similarly, the masses of the tablets did not differ significantly between the batches (Table 6).

**Table 6: dimension and mass of printed tablets**

| | **side a [mm]** | **side b [mm]** | **side c [mm]** | **mass [mg]** |
|---|---|---|---|---|
| **batch 1** | 10.875 | 10.771 | 5.609 | 318 |
| | 10.698 | 11.356 | 5.421 | 307 |
| | 10.665 | 10.994 | 5.286 | 285 |
| | 10.683 | 11.315 | 5.488 | 302 |
| | 10.817 | 10.794 | 5.65 | 334 |
| | 11.079 | 10.653 | 5.597 | 335 |
| | 10.758 | 10.859 | 5.007 | 280 |
| | 10.726 | 10.995 | 5.366 | 312 |
| | 10.52 | 10.806 | 5.434 | 336 |
| | 10.489 | 10.906 | 5.47 | 332 |
| | 10.165 | 10.702 | 5.394 | 299 |
| | 10.603 | 10.681 | 5.315 | 288 |
| **average** | 10.67 | 10.9 | 5.42 | 310.67 |
| **standard deviation** | 0.23 | 0.23 | 0.17 | 20.51 |
| **batch 2** | 10.293 | 10.457 | 5.492 | 334 |
| | 10.592 | 10.355 | 5.667 | 348 |
| | 10.499 | 9.761 | 5.557 | 347 |
| | 10.361 | 10.118 | 5.254 | 319 |
| | 10.157 | 10.482 | 5.488 | 351 |
| | 9.865 | 10.405 | 5.475 | 327 |
| | 10.511 | 10.516 | 5.644 | 329 |
| | 10.654 | 10.307 | 5.607 | 333 |
| | 10.779 | 10.196 | 5.748 | 346 |
| | 10.827 | 10.276 | 5.388 | 338 |
| | 10.292 | 10.535 | 5.523 | 350 |
| | 10.639 | 9.587 | 5.367 | 317 |
| **average** | 10.46 | 10.25 | 5.52 | 336.58 |
| **standard deviation** | 0.28 | 0.3 | 0.14 | 11.98 |
| **batch 3** | 10.417 | 10.358 | 5.5861 | 334 |
| | 10.276 | 10.703 | 5.852 | 316 |
| | 10.482 | 10.222 | 5.866 | 312 |
| | 10.41 | 10.79 | 5.675 | 319 |
| | 10.202 | 10.544 | 5.805 | 305 |
| **average** | 10.36 | 10.52 | 5.81 | 317.2 |
| **standard deviation** | 0.11 | 0.24 | 0.08 | 10.76 |

### 5.2.2 Test of the drug load capacity

To test the loading capacity with an active ingredient simple glass beads, Spheriglass 2429, were used as model substance. The matrix forming agency was again Spherolac 100 AH (<180 µm). A load capacity up to 50 wt% was possible, without delaying the speed of hardening (in case of printing with stable α-lactose anhydrate). Due to the very fast consolidation process, the printed tablets could be handled immediately after the printing process is finished. Different shapes and sizes and therefore different contents of the model substance could be printed. A new approach is the printing of continuous tablets, which can then be broken off to single doses.

### 5.2.3 Tablet porosity

The porosity, ratio between the sample void volumes and its external volume, of a tablet should be high if a fast disintegration is desired (e.g. for sublingual tablets). Porosity was on one hand measured using mercury porosimetry and on the other hand calculated theoretically. For a complete mercury intrusion porosimetry (MIP) measurement, two different porosimeter types are needed - a low-pressure porosimeter (LP-MIP) and a high-pressure porosimeter (HP-MIP). As the LP-MIP, Pascal 140 (Thermo Fisher Scientific, I-Milano) was used and the Porosimeter 2000 (Carlo Erba, I-Milano) as the HP-MIP.

Pure Spherolac 100 was used for the experiments. One tablet was printed by the 3D-powder printer, with Spherolac 100 AH as starting substance, and one tablet was pressed with a tablet press (10 kN). The substance of the pressed tablet was Spherolac 100 MH. The calculations resulted in a porosity of 17.7 % for the compressed tablet and 71.3 % for the printed tablet.

Mercury porosimetry of the 3D-powder printed tablet indicated a porosity of 63.8% and an average pore radius of 48 Micron. The measured porosity is lower than the calculated value (71.3%), because large pores were already covered by mercury before the measurement started. Thus, the porosity of the printed tablet is much higher than the porosity of the pressed tablet and guarantees a fast intrusion of fluids into the tablet and a fast disintegration.

## Claims

1. Process for the preparation of a dosage form comprising a fast consolidating compound and an active ingredient, comprising the steps of:
(a) providing a mixture of at least one fast consolidating compound and at least one active ingredient;
(b) adding at least one solvent to the mixture of step (a) to obtain a different solid form (cross-linking phase) of the fast consolidating compound; and
(c) allowing the solid form of the fast consolidating compound of step (b) to consolidate to form the dosage form.

2. Process for the preparation of a consolidated composition, comprising the steps of:
(a) providing at least one fast consolidating compound;
(b) adding at least one solvent to the fast consolidating compound of step (a) to obtain a different solid form (cross-linking phase) of the fast consolidating compound; and
(c) allowing the solid form of the fast consolidating compound of step (b) to consolidate,
wherein the solid form consolidates in less than 5 minutes.

3. The process according to claim 1 or 2, wherein the fast consolidating compound is an organic or inorganic salt, carbohydrate, or an amino acid.

4. The process according to claim 3, wherein the carbohydrate or the salt is a partially dehydrated hydrate or an anhydrous form of α-lactose, raffinose, glucose, lactitol, calcium lactate, MgSO₄, Na₂SO₄ or Mg citrate.

5. The process according to any one of claims 1 to 4, wherein the solvent is selected from water, alcohols, ether, esters, ketones, hydrocarbons, dimethylsulfoxide or a mixture thereof.

6. The process according to any one of claims 1 to 5, further comprising a binder, a flavoring agent, a colorant, a filler, a nutrient, a mineral supplement or a mixture thereof.

7. The process according to claim 6, wherein the binder is selected from the group consisting of polyethylene glycol, polyvinylpyrrolidon, polyvinylalcohol, or a mixture thereof.

8. The process according to any one of claims 1 or 3 to 7, wherein the active ingredient is a food supplement or a pharmaceutically active ingredient or a mixture thereof.

9. The process according to claim 8, wherein the pharmaceutically active ingredient is selected from the group consisting of an analgesic, a cholesterol-lowering medicament, an agent for treating hypertension, an agent for treating diabetes, an agent for treating cardiac conditions, an antihistamine, a hormone-containing drug, an antiepileptic or a mixture thereof.

10. Dosage form obtainable by the process according to any one of claims 1 or 3 to 11.

11. The dosage form of claim 10, wherein the dosage form is a tablet, capsule, lozenge, pastille, granule, sachet, reconstitutable powder, powder, dry powder inhaler or a chewable.

12. Use of a mixture comprising at least one fast consolidating compound and at least one solvent for 3D printing or a moulding process.

13. The use of claim 12, wherein the fast consolidating compound is an organic or inorganic salt, a carbohydrate or an amino acid, and/or the solvent is selected from water, alcohol or a mixture thereof.

14. The use of claim 12 or 13, wherein the mixture further comprises an active ingredient.

15. The use of claim 14, wherein the active agent is selected from the group consisting of an analgesic, a cholesterol-lowering medicament, an agent for treating hypertension, an agent for treating diabetes, an agent for treating cardiac conditions, an antihistamine, a hormone-containing drug, an antiepileptic or a mixture thereof.
